# EUROPEAN PATENT APPLICATION

(11) **EP 4 299 584 A1**
(43) Date of publication of application: **03.01.2024**
(21) Application number: 22182352.9
(22) Date of filing: 30.06.2022
(51) Int. Cl.: C07K 14/705, A61P 35/00

(54) **MUTANT OESTROGEN RECEPTOR LIGAND BINDING DOMAINS**

(71) Applicant: Katholieke Universiteit Leuven KU Leuven Research & Development, 3000 Leuven (BE); VIB VZW, 9052 Gent (BE)
(72) Inventor: The designation of the inventor has not yet been filed

(57) **Abstract**

The invention relates to polypeptides comprising the ligand binding domain of an oestrogen receptor, wherein the polypeptide comprises, with reference to the amino acid sequence of human oestrogen receptor isoform 1, a mutation in the ligand binding domain at position 539 and 543.

## Description

### FIELD OF THE INVENTION

The invention relates to inducible switches for controlling gene expression.

### BACKGROUND OF THE INVENTION

Nuclear receptors (NRs) form a large ligand-sensing gene family of proteins that modulate the development, homeostasis and metabolism of metazoans. Upon ligand binding, the NRs undergo an activating conformational change leading up to the regulation of cellular gene expression programs¹. NRs have been applied as gene expression switches and regulatory domains in synthetic biological applications, for instance, acting as biosensors for environmental toxins^{2, 3}, as switches in non-native metazoans to provide synthetic control over cellular function^{4, 5} or as regulatory domains of genomic tools⁶⁻⁸. Nevertheless, the use of NR-based tools is compromised because their cognate ligands interfere with the native NR actions or are found detrimental for the host organism⁹⁻¹¹.

*De novo* NRs (XRs) that employ chemicals of choice will overcome these limitations thereby expanding their scope and utility. However, the stringent coupling between ligand recognition and transcriptional regulation have proven challenging to engineering NRs towards designer inducers. Target residues for accommodating de *novo* ligands are unknown and can be distributed throughout the ligand binding pocket but also the ligand-binding domain^{12, 13}. Furthermore, ligand-binding domain substitutions should preserve the functional interactions with the necessary coregulators¹⁴.

Previous work demonstrated the success of random mutagenesis by developing an ERα-LBD variant (ERT2) that enables nuclear translocation upon binding of tamoxifen, the well-known ERα antagonist used in the treatment of breast cancer¹⁵⁻¹⁷. However, despite successive saturation mutagenesis rounds and structural- as well as dose optimization strategies^{8, 18}, the ER mutants remained driven by physiological levels of the oestrogen ligand and the high tamoxifen levels required for activation, interfere with endocrinology^{19, 20}. The necessary further optimization of functional tamoxifen receptor proteins is hampered because of the challenging nature of the rational protein design^{12, 21, 22}. Alternatively, large-scale mutagenesis strategies via directed evolution²³ on the other hand can provide extensive libraries, but lack high-throughput metazoan profiling platforms. However, recent advances in synthetic biology and genetic engineering have expanded the applications of directed evolution for the engineering of heterologous proteins to *Saccharomyces cerevisiae*^{24- 26}.

Conventional rational protein design strategies employ the hit-or-miss principle²² and traditional directed evolution requires isolation of the marginal fraction of desired variants.

### SUMMARY OF THE INVENTION

Nuclear receptors and their ligand-binding domains are applied in synthetic biology to control activity of genomic tools like CRE recombinase and Cas proteins. Application in mammalians needs further optimization because of the interference of the activating ligands with general biology. By combining rational protein design with directed evolution in Saccharomyces cerevisiae, we established a strategy for altering NR ligand specificity. This enabled evolving the oestrogen receptor alpha into the TERRA variant (L391T/L539F/M543E), which is activated by tamoxifen as its primary agonist (EC50: 112 nM). In comparison with the ERa, the TERRA variant was induced by lower tamoxifen and higher E2 levels in mammalian cells. When the TERRA LBD was fused to Cas9 and Cre, they became more sensitive to tamoxifen in mammalian cells. This platform will enable the development of de novo ligand inducible switches for synthetic biology in mammalian cells and will enable landscaping of NR-related drug resistance f.e. in breast and prostate cancer.

Here, we present a so-called rational evolution as a strategy that combines mutagenesis of multiple *in silico* defined amino acid locations with high-throughput profiling of stably integrated XR variants in S. *cerevisiae.* This strategy maintains a targeted directed evolution of amino acid locations assigned through rational protein design. This provides the ability to selectively mutagenize multiple distant hotspot amino acid locations thus propagating the portion of desired variants. The XR library is integrated using CRISPR-Cas9, thereby ensuring that every transformant expresses only a single XR variant. A subsequent strong phenotype-genotype linkage facilitates diverse screening and selection methodologies for the identification of mutants with desired phenotypes.

This study describes how we altered the ligand preference of the human oestrogen receptor α to a chemical of choice using rational evolution in S. *cerevisiae.* The mutant phenotypes are transferable across mammalian species and can be further converted into *in vitro* and *in vivo* genome editing tools.

The invention is further summarised in the following statements:
1. A polypeptide comprising the ligand binding domain of an oestrogen receptor, characterised in that the polypeptide comprises, with reference to the amino acid sequence of human oestrogen receptor isoform 1 [SEQ ID NO: 55], a mutation in the ligand binding domain at position 539 and 543.
   The reference to the sequence of human oestrogen receptor isoform 1 is merely to indicate the nature and position of mutated amino acids. The claimed polypeptides thus equally relate to polypeptides such as other isoforms of human oestrogen receptor, oestrogen receptors from other species than human, fragments of an oestrogen receptor comprising the ligand binding domain, fusion proteins comprising the ligand binding domain, and sequences having, apart from the claimed mutations, a sequence which is 80, 85, 90, 95, 97, 98 or 99 % identical to the sequence the ligand binding domain (amino acids 310 to 547) of SEQ ID NO:55.
2 The polypeptide according to statement 1, which further comprises with reference to the amino acid sequence of human oestrogen receptor isoform 1 [SEQ ID NO: 55], a mutation at position 391.
3. The polypeptide according to statement 1 or 2, wherein the mutation at position 539 is L539F, L539Y or L539W.
4. The polypeptide according to any one of statements 1 to 3, wherein the mutation at position 539 is L539F.
5. The polypeptide according to statement 1 or 2, wherein the mutation at position 543 is M543E, M543D, M543Q, or M543N.
6. The polypeptide according to statement 1, 2 or 5, wherein the mutation at position 543 is M543E.
7. The polypeptide according to statement 1 or 2, wherein the mutation at position 391 is L391T or L931S.
8. The polypeptide according to statement 1, 2 or 7, wherein the mutation at position 391 is L391T.
9. The polypeptide according to any one of statements 1 to 8,
   wherein the mutation at position 539 is L539F,
   wherein the mutation at position 543 is M543E,
   wherein the mutation at position 391 is L391T.
10. The polypeptide according to any one of statements 1 to 9 which is a fusion protein with a cre-recombinase or a Cas protein.
11. A polynucleotide encoding a polypeptide according to any one of statements 1 to 10.
12. A method of generating modified versions of the ligand binding domain (LBD) of a steroid receptor comprising the steps of:
   a) introducing a first set of amino acid mutations at one or more of the amino acids corresponding to the amino acids L525, L539, L540, M543, and L544 of the human oestrogen receptor isoform 1 amino acid sequence [SEQ ID NO: 55],
   b) testing a polypeptide comprising the LBD modified in step b) for activation by a compound other than its natural ligand, and selecting mutants which are activated by said compound at an EC50 below 1 µM, or below 500 nM,
   c) further introducing a second set of amino acid mutations at one or more of the amino acids corresponding to the amino acids L384, L387, M388, L391, G400 of the human oestrogen receptor isoform 1 amino acid sequence [SEQ ID NO: 55],
   d) testing a protein comprising the LBD further modified in step c) for activation by its natural ligand, and selecting mutants which are activated by said natural ligand with an EC50 above 10 nM, above 25 nM, above 50 nM or above 75 nm, or selecting mutants which are activated by said natural ligand at an EC50 which is at least 5, 10, or 20 times higher compared to EC50 obtained by said natural ligand with a non-modified LBD.
   Other suitable receptors for the methods of the current invention are androgen receptors, mineralocorticoid receptor, glucocorticoid receptor, oestrogen receptor beta, and oestrogen related receptors. Alignment these protein sequences with SEQ ID NO: 55 allows to identify the relevant amino acids in these other receptors.
13. The method according to statement 12, wherein the EC50 is determined by measuring in a cell the expression level of a steroid dependent reporter gene.
14. The method according to statement 12, wherein the activity is determined by measuring the fluorescence of a fluorescent reporter protein.
15. The method according to statement 12, wherein the activity is determined by measuring the enzymatic activity of a reporter enzyme.
16. The method according to statement 12, wherein the steroid receptor is the oestrogen receptor, and the natural ligand is oestradiol.
17. The method according to statement 12, wherein said compound is tamoxifen.
18. The method according to statement 12, wherein the LBD in step c) used for introducing the second set of mutations comprises mutations at position 539 and 543.
19. The method according to statement 12, wherein the LBD in step c) used for introducing the second set of mutations comprises, with reference to the amino acid sequence of human oestrogen receptor isoform 1 amino acid sequence [SEQ ID NO: 55], at position 539 the mutation L539F, L539Y or L539W, and comprises at position 543 the mutation M543D, M543E M543Q, or M543N.
20. The method according to statement 12, wherein the LBD in step c) used for introducing the second set of mutations comprises, with reference to the amino acid sequence of human oestrogen receptor isoform 1 amino acid sequence [SEQ ID NO: 55], at position 539 the mutation L539F, and comprises at position 543 the mutation M543E.

### DETAILED DESCRIPTION

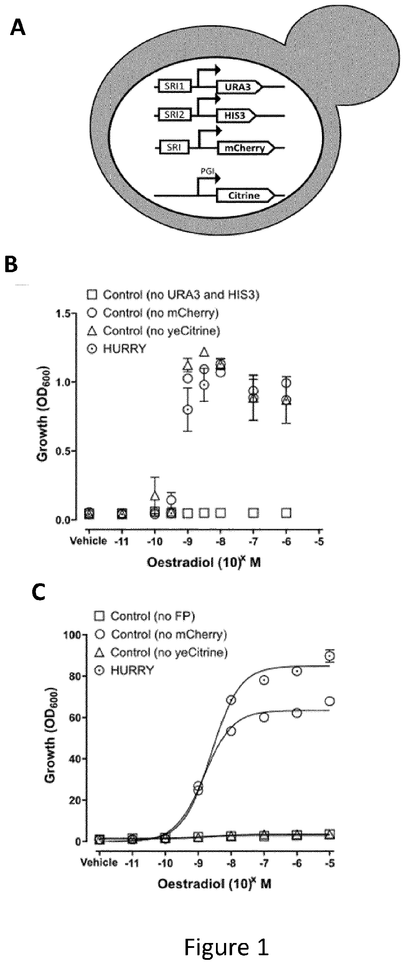

Figure 1: Saccharomyces cerevisiae platform for evolving Nuclear receptors towards designer chemicals. (A) The Hurry reporter strain with encompassing the dual survival and fluorescent reporter cassettes under NR control. (B) Oestrogen responsive growth curves of the S. cerevisiae strains modified with the indicated yERα controlled reporters. (C) Dose response curve of the yERα controlled RFP reporter in the modified S. cerevisiae strains The error bars indicate the standard error of the mean of n = 3 (SEM).

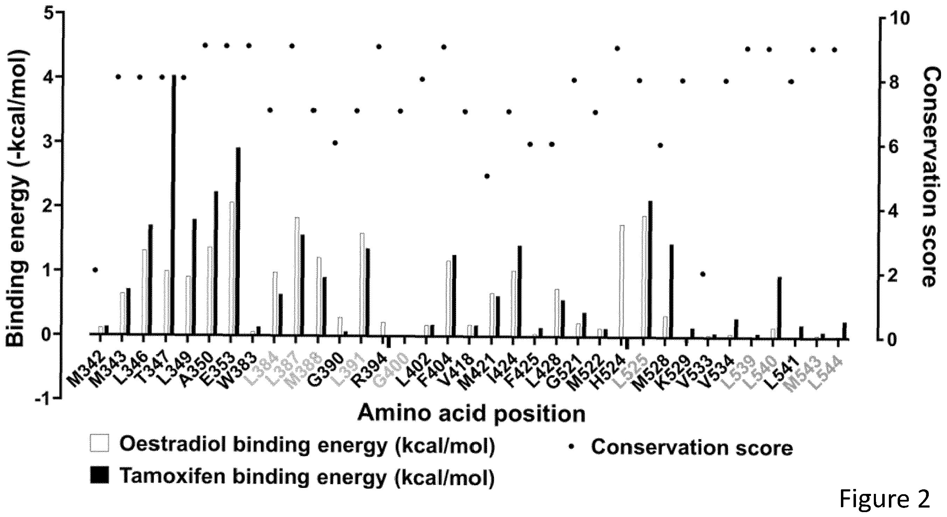

Figure 2: Amino acid conservation and contribution to the ligand interface of the human oestrogen receptor α with oestradiol or tamoxifen, pinpointing conserved regions and highlighting promiscuous amino acids for rational evolution.

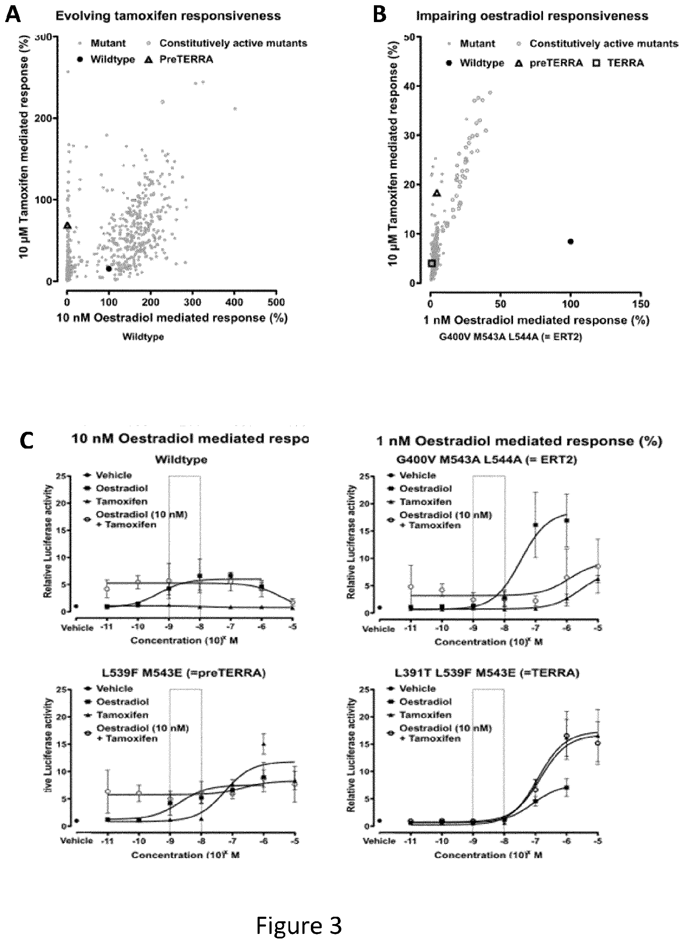

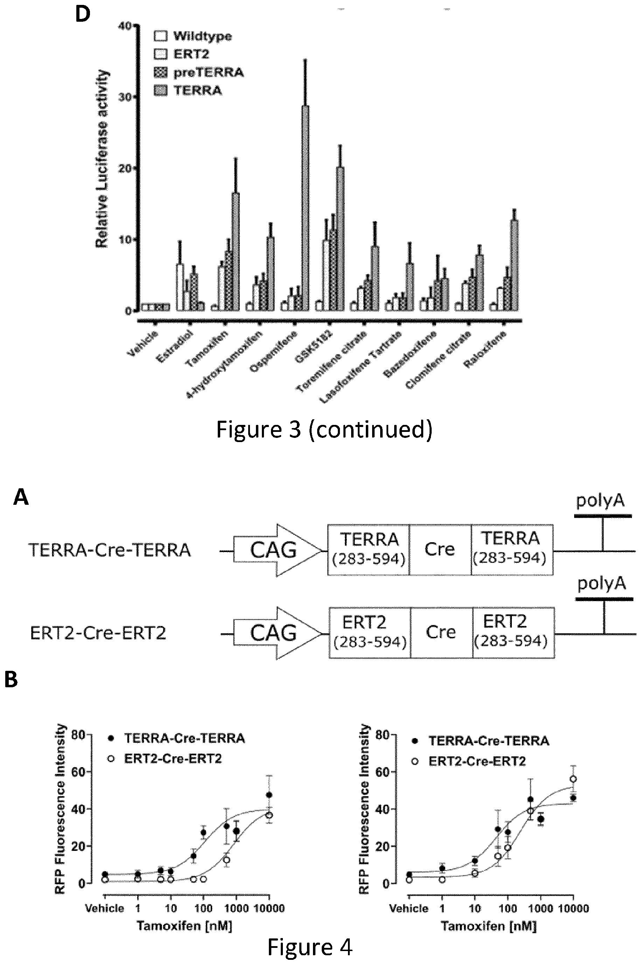

Figure 3: Rational evolution method for steering the oestrogen receptor α (ERα) towards the designer chemical, tamoxifen. (Characterization of rationally evolved NR variants in the HURRY strain for their respective oestradiol and mediated activities. (B) Evaluation of the rationally evolved top isolates in mammalian cells for their respective oestradiol, tamoxifen and combined activities. Physiological oestrogen concentrations are depicted in grey. (C) PreTERRA derived NR variants, accommodating mutations aimed at impairing oestradiol binding, profiling in the HURRY strain. (D) Mapping the influence of tamoxifen analogues on the wildtype (ERα), current tamoxifen inducible ERα (ERT2), preTERRA and the TERRA mutant.
Figure 4: Conversion of the TERRA variant into common genomic tools. (A) Dual tamoxifen inducible Cre recombinase expression cassette composed of the CAG promotor, the Cre recombinase CDS fused both up- and downstream by either the TERRA-LBD or the ERT2-LBD and the poly A signal. (B) Dose-response curve of the TERRA-Cre-TERRA and ERT2-Cre-ERT2 fusion proteins for oestradiol and tamoxifen after stimulation for 24 hours in mammalian HEK-LoxP cells. Error bars represent the standard error of the mean (n=3).

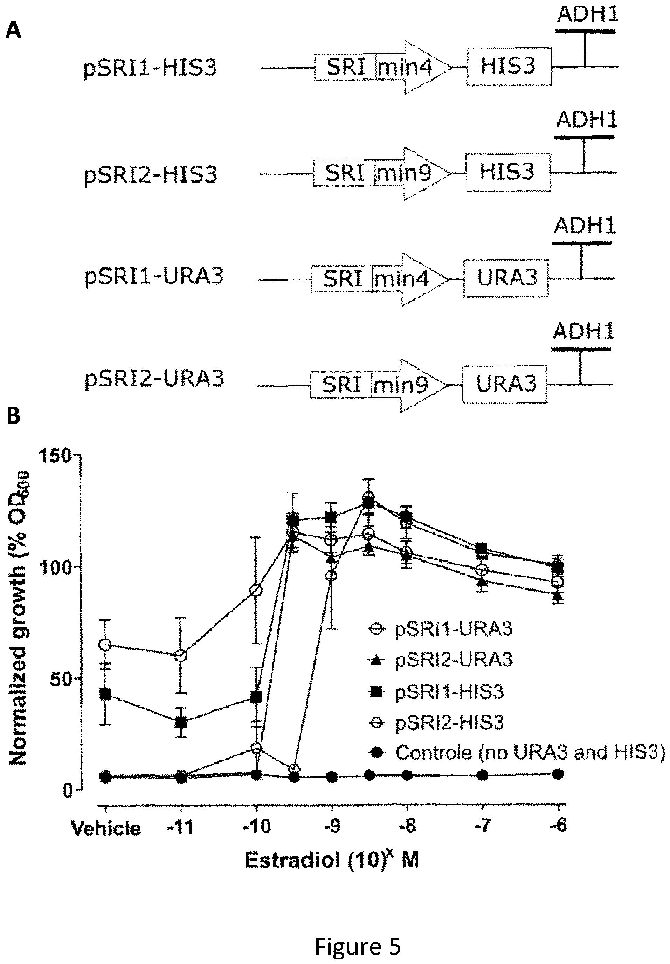

Figure 5: Construction of growth dependable NR reporter cassettes in Saccharomyces Cerevisiae. (A) Reporter cassettes are composed of the SRi enhancer region and respectively synthetic core promotor 4 or 9 (min4 or min9), rendering pSRI1 and pSRI2. The pSRI-HIS3 or pSRI-URA3 reporters were integrated in respectively the HIS3 or URA3 loci in the native BY4741 genome. (B) The NR dependency of the pSRI promotors was evaluated by the stable integrated yERα in the ARS208a locus and stimulation by oestradiol. Error bars represent the standard error of the mean (n=3).

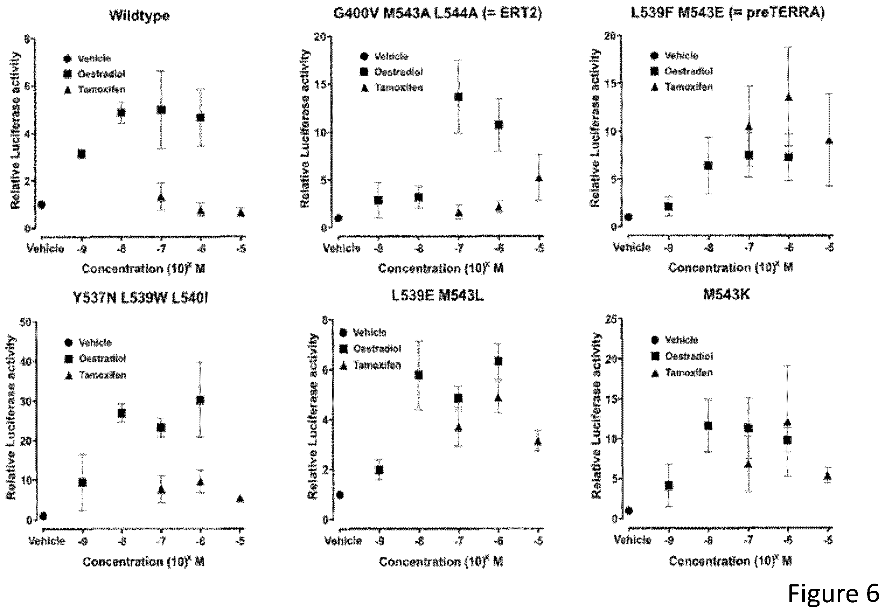

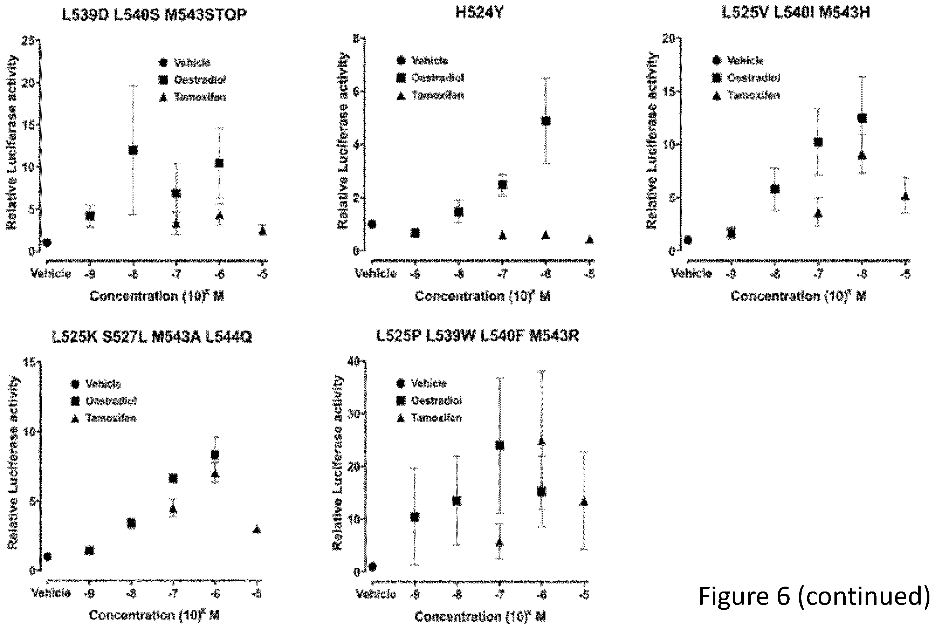

Figure 6: Mammalian cell line evaluation of the human codon-optimized nine most tamoxifen selective isolates obtained after rationally evolving the L525, L539, L540, M543 and L544 amino acid positions of the wildtype oestrogen receptor α. Error bars represent the standard error of the mean (n=3).

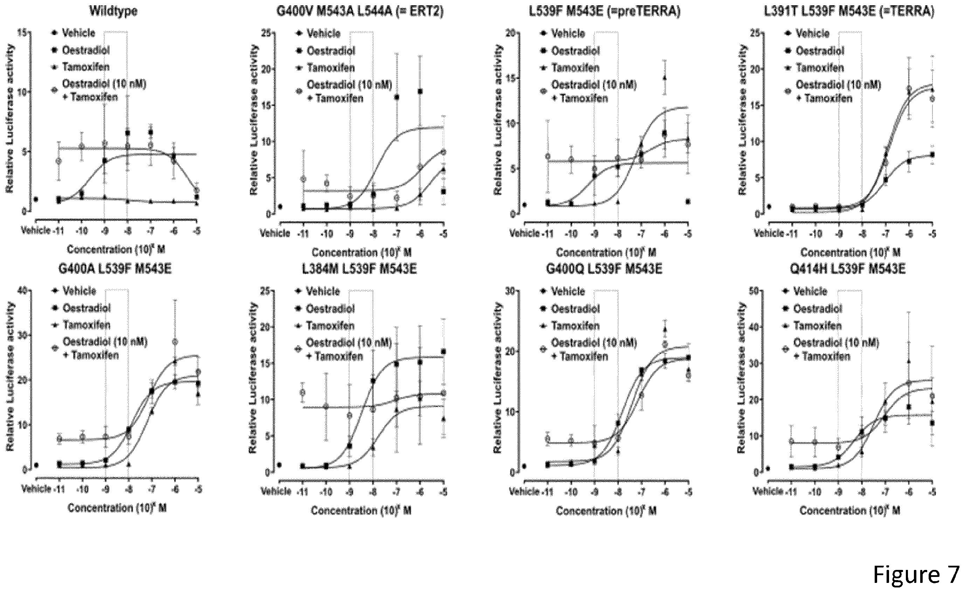

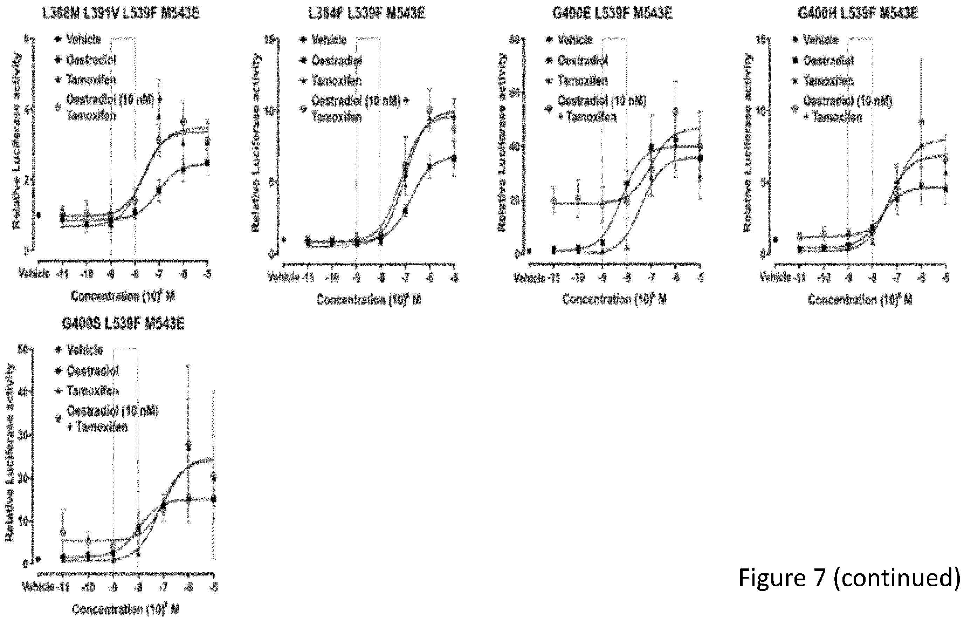

Figure 7: Mammalian cell line evaluation of the human codon optimized ten most tamoxifen selective isolates obtained after rationally evolving the L384 L387 M388 L391 and G400 positions of the preTERRA variant. Error bars represent the standard error of the mean (n=3).

Modifying and understanding the ligand preference of NRs is of considerable interest in the fields of biotechnology, given their prevalence in eukaryotic gene editing tools and in medicine, where therapeutic failure can be connected to cancer progression and drug resistance. However, designing functional *de novo* NR (XR) proteins responding to chemicals of interest has proven challenging.

Our results provide a S. *Cerevisiae* platform and general strategy that combines targeted directed evolution with rational protein design predictions to evolve any NR towards inducers of interest (Figure 2). As proof-of-principle, the human oestrogen receptor α was evolved towards tamoxifen, the ERα antagonist. After a single evolution round, multiple variants were obtained that exhibited pronounced tamoxifen mediated activities in both yeast and mammalian cells (Figure 3). The top isolate from this first round (preTERRA, L539F M543E) demonstrated an EC50 of 56 nM for tamoxifen. Nonetheless, oestradiol remained the potent inducer of the preTERRA variant (EC50 of 0.54 nM), comparable to the wildtype ERα (EC50 of 0.23 nM). Further evolving the preTERRA to disrupt activation by oestradiol yielded the TERRA mutant (L391T L539F M543E). The TERRA mutant retains the tamoxifen mediated phenotype (EC50 of 122 nM) but is no longer induced by physiological levels of oestradiol (EC50 of 91 nM). As illustrated, the TERRA-LBD can be used as regulatory domain upon fusion with Cre recombinase or Cas9 both *in vitro* and *in vivo.* The subtoxic tamoxifen levels can mediate the nuclear translocation and require little to none assay optimization compared to the current ERT2 system.

Further comparison of the TERRA, preTERRA, ERT2 and the wildtype ERα regarding their responses to tamoxifen analogues displayed a more pronounced induction by the TERRA mutant in all cases. The underlying mechanism behind this has been attributed to a novel hydrogen bond formation between L391T and the phenol of tamoxifen (or analogue). The L539F M543E demonstrate a distortion in helix 12 positioning the D538 in proximity of tamoxifen's amine group, enabling another hydrogen bond to form.

Despite the improved sensitivity towards subtoxic amounts of tamoxifen, safety precautions are eminent when handling tamoxifen³¹. The next step should aim at either synthesizing a tamoxifen analogue that does not hamper the native endocrine system or intend to evolve NRs towards chemicals that show no influence on native NRs. Both approaches can mature towards an orthogonal regulatory domain. Altering the ligand preference of NRs towards target ligands distinct from the natural ligands require unique protein-ligand interfaces that also maintain the overall protein architecture and signalling mechanism. *De novo* computational protein designs may be fruitful but suffer from the protein structure prediction problem³² resulting in limited success rates^{12, 21}, combinatorial mutagenesis of putative amino acid locations on the other hand allows natural selection to isolate the desired phenotypes. The plasticity and residue conservation of every NR varies and necessitate a case-by-case identification, randomisation and characterization of putative residues. Once initial XR hits are found, their structure can be further optimized to increase the level of activity and selectivity. Every XR-ligand pair can be converted in *in vitro* and *in vivo* gene expression switches, genome editing tools or biosensors and propagated across species. An expanded repertoire of various evolved XR variants with separate inducers enable tuneable, independent control of multiple genes in signalling cascades.

From *in silico* model to metazoan genome editing tool, the rational evolution methodology can be converted to other ligand sensing proteins such as riboswitches³³ and allosteric transcription factors³⁴, if the appropriate reporter gene circuits and the protein structures are available.

Besides biological tools, the NRs are considered major therapeutic targets for the treatment of various diseases such as cancer and inflammation. By engineering the NR towards a particular therapeutic, the rational evolution set-up can landscape NR-related drug resistance. Connecting escape mutant landscapes of drug candidates to individual genetic tumour profiles will give insight in providing the appropriate treatment for every patient.

### EXAMPLE 1: Saccharomyces cerevisiae platform for evolving nuclear receptors

To ensure qualitative and quantitative readouts for NR activity in S. *cerevisiae,* we engineered the HURRY strain with gene circuits relating NR activity to survival and fluorescence (Figure 1a). Replacing the native URA3 and HIS3 promotors with universal steroid receptor enhancer regions²⁷ and synthetic core promotors²⁸ rendered its growth dependent on NR activity (Figure 5). The combination of two growth selective pressures promotes the evolutionary stability when introducing or characterizing NR variants²⁹. By including an mCherry reporter, this HURRY strain further permits fluorescence-based profiling of NR activity. Validation of NR dependent growth and NR activity read out (Figure 1b-c) was mediated by the S. *cerevisiae* codon-optimized ERα (yERα) upon integration in the ARS208a locus. This promoted recombinant protein expression as well as genomic integration efficiency³⁰. The oestradiol response profile for yERα in the HURRY strain were similar to the human ERα (hERα) in human cells with EC50 of 2.5 nM (Figure 1 and Table 1). Taken together, the yERα simultaneously induces expression of the two growth selective pressure cassettes as well as the fluorescence reporter gene in the S. *cerevisiae* HURRY strain. We therefore took the yERα as a starting point for the development of a directed evolution screening strategy for changing the ERa towards a synthetic tamoxifen receptor using the HURRY strain as a platform.

**Table 1: EC50s of the receptor variants for oestradiol and tamoxifen**

| | **EC50 in HEK 293 cell lines** | |
|---|---|---|
| | *Oestradiol* | *Tamoxifen* |
| **Wildtype** | 0,47 nM | 12,44 nM^{∗} |
| **ERα** | | |
| **ERT2** | 3,37 nM | 2 615 nM |
| **preTERRA** | 2,06 nM | 56 nM |
| **TERRA** | 83,28 nM | 122 nM |

| | | |
|---|---|---|
| ^{∗}*Tamoxifen acts as an antagonist on the Wildtype ERα in HEK 293 cells (value represents the IC50)* ^{∗∗}*Values are based on non-linear regression (PRISM 9.3.1)* | | |

### EXAMPLE 2: Rational evolution methodology

We designed a rational evolution methodology that allows to mutate multiple residues simultaneously and detect receptor activity in the yeast HURRY strain. Iterative rounds can result in a variant that is optimized for recognition of a ligand with optional counterselection rounds against the natural ligand of the ER. Our experimental pipeline is divided in three sections: *in silico* identification of putative amino acid (AA) locations which could be involved in the adoption of the new ligand, simultaneous diversification of these AA positions and characterization of the variant library. Here we illustrated the rational evolution of NRs towards the chemicals of choice using the ERα as a test case. To identify putative AA locations, every AA of the ERα ligand binding domain (LBD), was defined by their evolutionary conservation scores among NRs and relative binding energies towards the native ligand oestradiol, or the synthetic ER partial agonist tamoxifen. Residues with low conservation scores and high binding affinities with oestradiol (Figure 2b-c) were subjected to further diversification.

Five promiscuous AA codons were simultaneously diversified in all possible AA coding triplet combinations by using the NNK-motif. By introducing sequence homology to the S. *cerevisiae* ARS208a locus both up- and downstream of the variant in the pool, they were integrated in the HURRY strain by means of CRISPR-Cas9, thereby ensuring every transformant to express only one XR variant. The mutants with improved tamoxifen selectivity were selected by means of cell sorting based on mCherry fluorescence intensity. The top mutant will becomes the new starting point for *in silico* defining the subsequent residues to optimize, followed by successive rounds of diversification and characterization.

### EXAMPLE 3: Evolving tamoxifen selective receptors

The proposed rational evolution methodology was applied to the evolution of the ERα towards tamoxifen as agonist. Residues L525, L539, L540, M543 and L544 were simultaneously changed into all possible amino acid combinations. This was done by using the NNK-motif in the open reading frame of ER. To ensure that every mutant out of the 3.36 × 10⁷ variant library was screened at least 15 times, 60 variant pools were independently introduced via CRISPR library integration in the HURRY strain, leading to approximately 5.03 × 10⁸ independent yeast transformants. After selection of this yeast library on tamoxifen selective media, transformants were sorted based on their tamoxifen-induced fluorescence. This generally resulted in collecting the top 2-3% of the tamoxifen induced yERα mutant libraries. Across six independent FACS-isolated cell batches, a total of 960 colonies were clonally tested for their respective oestradiol and tamoxifen stimulation responses. These isolates were subsequently ranked according to their response to tamoxifen and oestradiol (Figure 3a). The top 20 individual clones from the following four categories were sequenced: (1) tamoxifen selective, (2) oestradiol selective, (3) dual activity with high tamoxifen induction (4) dual activity with high oestradiol induction (Tables 2-5).

The top nine, unique tamoxifen selective colonies were codon-optimized for evaluation in mammalian cells (Figure 6). Eight out of nine receptor variants, demonstrated tamoxifen responsive reporter gene induction, illustrating the conversion of tamoxifen into an agonist for these variants even in mammalian cells. In mammalian cells, the L539F M543E mutant (preTERRA) established the highest tamoxifen sensitivity and maximal activity at 10 µM (Table 1). Compared to the current tamoxifen inducible system (ERT2), preTERRA shows a 46-fold higher tamoxifen sensitivity and twofold in maximal activity and an absence of any basal transactivation (Figure 3b). However, like ERT2, the preTERRA mutant is still responsive to physiological oestradiol levels (1-10 nM) limiting its possible translation to metazoan synthetic biological tools. Further optimization was therefore aimed at preventing oestradiol binding.

Via computational methods, L384 L387 M388 L391 and G400 were identified as essential AA locations for oestradiol binding in the preTERRA variant. The NNK-motif was used to simultaneously randomize the five denoted positions in all possible AA combinations, rendering the 3.36 × 10⁷ preTERRA variant pool. Again, up to 60 variant pools were independently introduced via CRISPR library integration in the HURRY strain aiming at 5.03 × 10⁸ separate transformants in total. The transformants were pooled, cultivated in medium supplemented with 10 µM Tam and sorted via FACS based on the mCherry reporter expression. Upon clonally evaluating 960 sorted transformants to E2 (100 nM) and Tam (10 µM), we observed most variants had lower E2 and Tam responsiveness compared to the preTERRA variant, 36 clones demonstrated a higher Tamoxifen responsiveness (Figure 3c).

We selected the top 30 tamoxifen selective variants indicated in Fig 3 panel E with a selectivity ratio for E2 over Tam ranging from of 2.5 to 27. Genotyping retrieved the preTERRA mutant or a synonymous variant in 16 cases (Table 6). This observation suggests that the maximal tamoxifen response in yeast was already reached after the first rational evolution round. The 14 remaining transformants contained at least one non-synonymous mutation.

Next, mammalian codon optimized versions of the top ten unique tamoxifen selective variants were characterized in mammalian cells (Figure 7). All preTERRA derivatives retained the tamoxifen sensitivity and the majority (8 out of 10) displayed a reduced oestradiol sensitivity. Similar to the first evolution round, the rationally evolved top colony, named TERRA contains 1 additional mutation (L391T) compared to the preTERRA variant (L539F M543E). L391T retains the tamoxifen mediated phenotype of the preTERRA and reduces the oestradiol responses in yeast and mammalian cells. In contrast to ERT2 and preTERRA, the TERRA variant is no longer inducible by physiological oestradiol levels (1-10 nM), lacks basal transactivation and retains the pronounced tamoxifen selective behaviour (Figure 3b).

Further comparison of the TERRA, preTERRA, ERT2 and the wildtype ERα regarding their responses to tamoxifen analogues displayed a more pronounced induction for the TERRA mutant than for the wildtype ERα, ERT2 and preTERRA variants in all cases (Figure 3d).

### EXAMPLE 4: Conversion into in vitro genomic tools

The here rationally developed TERRA receptor overcomes the well-known limitations of NR-based synthetic tools in metazoans^{18, 31} by being insensitive to physiological oestradiol concentrations and tuneable by subtoxic tamoxifen levels. The TERRA receptor can immediately be applied in both metazoans and non-native model organisms as a synthetic transcription factor upon tamoxifen stimulation (Figure 3b). Moreover, we converted the TERRA variant into a regulatory domain to provide temporal control over common genomic tools, such as Cre recombinases and Cas9 endonucleases (Figure 4A). As established for the wildtype ERα and ERT2, binding of the LBD domain by an agonist leads to nuclear translocation, this nuclear shift can be used to switch on enzymes that require nuclear presence for their activity^{15, 16}. A dual regulatory domain design was used to prevent spontaneous translocation across the nuclear envelope in the reporter cell lines (Figure 4a)^{6, 8}. The ERT2-Cre-ERT2 system demonstrates tamoxifen- and oestradiol mediated translocation events with EC50 of respectively 1420 nM and 464 nM while the TERRA-Cre-TERRA system displayed recombination occurring at 96 nM tamoxifen and 41 nM oestradiol (Figure 4b).

### EXAMPLE 5: Adapting genomic tools for in vivo applications Structural insights in the TERRA variant

Insights in the structure-function relationship of the ERT2 and TERRA variants in complex with E2/Tam were revealed by the in silico model. While there is no clear indication attributing to the improved tamoxifen binding properties of the ERT2 mutant, the TERRA variant depicts clear protein-ligand interactions that support tamoxifen binding. The L539F and M543E mutations facilitate a distortion in the loop connecting helix 11-12 that displaces the negatively charged side chain of D538 towards the positively charged amine tail of tamoxifen. This electrostatic interaction enables the association with tamoxifen and its analogues. The hydroxyl group of the L391T mutation can facilitate the formation of a hydrogen bridge with the ketone functionality of tamoxifen and oestradiol. These tight molecular interactions attributed to the head and tail of tamoxifen form the foundation of the improved ligand binding characteristics of the TERRA variant towards tamoxifen and its analogues.

### EXAMPLE 6: methods and materials

### Cloning

### Expression vectors

The yeast expression vectors were derived from the p414-TEF1p-Cas9-CYC1t³⁵ (Addgene plasmid # 43802 ; http://n2t.net/addgene:43802 ; RRID:Addgene_43802). Via the In-fusion HD Cloning Kit (TaKaRa), the Cas9 coding DNA sequence (CDS) was substituted for the *Saccharomyces Cerevisiae* codon optimized ERα coding DNA sequence (yERα) provided with the N-terminal FLAG tag. Next, 120 bp homology with the ARS208a target site for rational evolution was introduced both upstream of the TEF1 promotor and downstream of the CYC1 terminator to yield the yERα template for rational evolution.

Mammalian expression vectors of the rationally evolved ERα mutants were established via site-directed mutagenesis of the FLAG tagged human ERα CDS (hERa) in the modified pEGFP-C1 plasmid²⁷. The pCMV-β-gal expression vector was acquired from Stratagene (La Jolla, CA).

Cre fusion constructs were generated in the pCAG-ERT2CreERT2 vector, a gift from Connie Cepko⁸ (Addgene plasmid # 13777 ; http://n2t.net/addgene: 13777 ; RRID:Addgene_13777), via restriction-ligation of the PCR amplified hTERRA ligand binding domain (LBD).

### Reporter constructs

Combining the universal steroid receptor enhancer region (SRi) with resp. the minimal synthetic core promotor (4 or 9)²⁸, the S. *cerevisiae* optimal Kozak sequence, the scHIS3 or scURA3 CDS and the ADH1 terminator, resulted in the SRi1-URA3 and SRi2-HIS3 reporters. The combination of the SRi enhancer region with the minimal CYC1 promotor, the yeast-enhanced mCherry CDS and the scDIT1 terminator yielded the SRi-mCherry reporter construct. The yemCherry CDS was isolated from the pGADT7-ADH700-yeCherry-pTEF1 (S.C.)-yeGFP-DHFR construct, a kind gift from Thomas Wandless³⁶ (Addgene plasmid # 24584 ; http://n2t.net/addgene:24584 ; RRID:Addgene_24584). The constitutively active yeast enhanced Citrine expression cassette was obtained via Gibson Assembly of PCR amplified PGI promotor, yeCitrine CDS and scCYC1 terminator.

The universal steroid receptor luciferase reporter (SRi-Luc) was used for profiling ERα activity in mammalian cells²⁷.

### CRISPR vectors

The CRISPR vectors were derived from a modified pV1382 vector. First, the pV1382³⁷, a gift from Gerald Fink (Addgene plasmid # 111436 ; http://n2t.net/ addgene:111436; RRID:Addgene_111436), was digested with BglII (Thermo Fisher Scientific) and self-ligated to remove the URA3 expression cassette. Next, the modified pV1382 vector was digested with BsmBI (Thermo Fisher Scientific) and dephosphorylated by shrimp alkaline phosphatase (Thermo Fisher Scientific). The linearized pV1382 was ligated with 5'-phosphorylated hybridized DNA primers, spanning the sgRNA sequence of interest and the required overhangs for ligation, to yield the CRISPR vectors for genome editing. sgRNA sequences were identified via CRISPOR³⁸.

### Adeno-associated viral designs

The AAV transfer plasmids were obtained via Gibson-Assembly of PCR amplified expression cassettes in the AAVpro^{®} packaging plasmid (AAV9) (TaKaRa). The pRC and pHelper plasmids (TaKaRa) were used for recombinant AAV production.

### Model organisms

### Saccharomyces Cerevisiae strain construction

The BY4741 S. *cerevisiae* (MATa *his3Δ1 leu2Δ0 met15Δ0 ura3Δ0*) strain was sequentially modified via homologous recombination and CRISPR-Cas9³⁹ to establish the HURRY strain (MATa *his3Δ1::[pSRI1-HIS3-tADH1] leu2Δ0 met15Δ0 ura3Δ0::[pSRI2-URA3-tADH1] hoΔ::[p(SRI-minCYC1)-mCherry-tADH1] can1::pPGI1-yeCitrine-tADH1*). *S. cerevisiae* codon optimized 3×FLAG-ERα was subsequently integrated in a similar fashion upstream of the ARS208a locus³⁰ in the HURRY strain (MATa *his3Δ1::[pSRI1-HIS3-tADH1] leu2Δ0 met15Δ0 ura3Δ0::[pSRI2-URA3-tADH1] hoΔ::[p(SRI-minCYC1)-mCherry-tADH1] can1::pPGI1-yeCitrine-tADH1 ars208a::pTEF1-3xFLAG-yERa-tCYC1*) for evaluating nuclear receptor activity. All integrations were verified by sequencing.

### Mammalian cell culturing

Human embryonal kidney 293T (HEK 293T) cells were obtained from the American Type Culture Collection (ATCC) while the human embryonal kidney 293 reporter strain (HEK293-loxP-GFP-RFP) was purchased from GenTarget Inc. Both cell lines were cultivated in Dulbecco's Modified Eagle Medium without phenol red (DMEM; Thermo Fisher Scientific) supplemented with GlutaMAX (Thermo Fisher Scientific), Penicillin-Streptomycin (Thermo Fisher Scientific) and 10% foetal calf serum (FCS; Thermo Fisher Scientific) or 5% charcoal-striped serum (CSS) for transfection experiments. Cells were incubated until 70-80% confluency in a 5% CO2 humidified 37°C incubator before subculturing.

### Mice

The mTmG mice were group-housed in the Animal Housing Facility of the KU Leuven under constant temperature, humidity and day/night cycle. Mouse had access to tap water and were fed *ad libitum* (1% calcium, 0.76% phosphate). Seven days before fluorescence imaging was done, mice were put on low fluorescent diet (2019 Teklad global 19% protein extruded, ENVIGO).

### Survival assay

From an overnight culture in synthetic complete medium (2% glucose), the cell number was determined by Cell Counter (Bio-Rad) and seeded at a density of 50.000 cells/ml in uracil and histidine depleted minimal medium spiked with the chemical of interest. Cells were incubated for 36h at 30°C on a shaking platform (750 rpm) before determining OD₆₀₀ by plate reader (Thermo Fisher).

### Rational evolution

### Identification

The ERα-LBDα (PDB ID:1QKU)⁴⁰ was taken as a reference for rational design. The full length amino acid sequence of human Oestrogen Receptor alpha [SEQ ID NO:55] is depicted below, and the numbering of this sequence is used as reference for the localisation of mutations. The residues surrounding the hormone binding pocket were selected for further evaluation. This consisted of a total of 24 positions. In addition, residues on helices 11 and 12 were also considered as a triple mutation in this region is shown to improve tamoxifen affinity with ERα¹⁵ Specific target positions were selected on the basis of their conservation scores and binding energy with oestradiol and tamoxifen. Conservation scores were obtained from ConSurf web-server⁴¹ and sequence logo was generated to determine the probability of naturally occurring amino acids. The binding energies were calculated by using snapshots from molecular dynamic simulations (GROMACS⁴²) followed by the Generalised Bonn approximation. For the first round of DE, we focused on increasing tamoxifen binding. Therefore, regions in vicinity of the tail of tamoxifen were chosen for directed evolution. We observed high probability of hydrophobic AAs at positions 525, 540 and 544 which are located around the amino group of tamoxifen which is unfavourable. Whereas, those at position 539 and 543 had fairly low binding energies with tamoxifen. Therefore, the above 5 positions were finally selected for site-directed mutagenesis. The best mutants obtained from round 1 still showed activity when induced with oestradiol. Therefore, in the next step, we aimed at reducing oestradiol binding and increasing specificity for tamoxifen. Residues present on helix 5 have higher binding energies and thus form stronger interactions with oestradiol than tamoxifen. In the structure of the binding pocket, residues at positions 384, 387, 388 and 391 are aligned towards the ligand and have fairly low conservation scores. Thus, these four positions were targeted for evolution towards tamoxifen

For the numbering of amino acids in the oestrogen receptor, reference is made to the below sequences.
SEQ ID NO :55
   Wild type sequence of human oestrogen receptor isoform 1. Mutations L539F, M543E and L391T are indicated below the sequence
   The ligand binding domain is underlined (amino acids 310 to 547] 
MTMTLHTKAS GMALLHQIQG NELEPLNRPQ LKIPLERPLG EVYLDSSKPA 50
VYNYPEGAAY EFNAAAAANA QVYGQTGLPY GPGSEAAAFG SNGLGGFPPL 100
NSVSPSPLML LHPPPQLSPF LQPHGQQVPY YLENEPSGYT VREAGPPAFY 150
RPNSDNRRQG GRERLASTND KGSMAMESAK ETRYCAVCND YASGYHYGVW 200
SCEGCKAFFK RSIQGHNDYM CPATNQCTID KNRRKSCQAC RLRKCYEVGM 250
MKGGIRKDRR GGRMLKHKRQ RDDGEGRGEV GSAGDMRAAN LWPSPLMIKR 300
SKKNSLALSL TADQMVSALL DAEPPILYSE YDPTRPFSEA SMMGLLTNLA 350
391
DRELVHMINW AKRVPGFVDL TLHDQVHLLE CAWLEILMIG LVWRSMEHPG 400
T
KLLFAPNLLL DRNQGKCVEG MVEIFDMLLA TSSRFRMMNL QGEEFVCLKS 450
IILLNSGVYT FLSSTLKSLE EKDHIHRVLD KITDTLIHLM AKAGLTLQQQ 500
 
539 543
HQRLAQLLLI LSHIRHMSNK GMEHLYSMKC KNVVPLYDLL LEMLDAHRLH 550
F E
APTSRGGASV EETDQSHLAT AGSTSSHSLQ KYYITGEAEG FPATV
SEQ ID NO:56
   Sequence flanking L391T mutation (mutated AA in bold)
   CAWLEILMIG TVWRSMEHPGK
SEQ ID NO:57
   Sequence flanking L539F and M543E mutation (mutated AA in bold)
   KCKNVVPLYD**F**LLE**E**LDAHRLHAPT
SEQ ID NO:58
   Fragment of Human oestrogen receptor depicting L539F, M543E and L391T (mutated AA in bold)

CAWLEILMIG TVWRSMEHPG KLLFAPNLLL DRNQGKCVEG MVEIFDMLLA
TSSRFRMMNL QGEEFVCLKS IILLNSGVYT FLSSTLKSLE EKDHIHRVLD
KITDTLIHLM AKAGLTLQQQ HQRLAQLLLI LSHIRHMSNK GMEHLYSMKC
KNVVPLYDFL LEELDAHRLH APT

### Diversification

The diversification strategy was adapted from the *in vitro* combinatorial mutagenesis method, Darwin Assembly²³. The purified yERα plasmid for rational evolution (300 ng/µl) was singularized by co-incubation with 30U of ExoIII (New England Biolabs) and 40U of Nt. BspQI (New England Biolabs) for 2h on 37°C. Next, the 5' phosphorylated spiked primers targeting the identified sites (44 pmol each) and the boundary primers (2.2 pmol each) assembled onto the singularized plasmid (0.22 pmol) by incubating for 1 hour at 50°C in 2x DA buffer (0.05 U/µl Q5 High-Fidelity DNA polymerase, 8 U/µl Taq DNA ligase, 2 mM NAD⁺, 0.4 mM of every dNTP, 10% (w/v) PEG 8000, 2 mM DTT, 1× CutSmart buffer). In parallel, 1 pmol Streptavidin coated beads (Dynabeads^{™} MyOne^{™} Streptavidin T1; Thermo Fishier Scientific) were blocked in 2x BWBS-T (20 mM Tris (pH 7.4), 2 M NaCl, 0.2% Tween-20, 2 mM EDTA) for an hour on a spinning wheel at room temperature. After isothermal assembly, the biotinylated DNA fragments were immobilized on the pre-blocked beads by incubation for 3h at room temperature on a spinning wheel. To release the immobilized DNA products, the beads were washed twice with warm NaOH (30 mM at 37°C) and once with EB-T (10 mM Tris (pH 8.8), 0.01% Tween-20, 0.1 mM EDTA) before resuspending in 10 µl EB (10 mM Tris (pH 8.8)). The resuspended beads were immediately used for PCR with outnest primers followed by gel purification with the GeneJet Gell extraction kit (Thermo Fisher Scientific). The purified linear mutant libraries are ready-for-use as repair template in the CRISPR-directed library transformation.

For the CRISPR-directed library integration, fifteen batches of the HURRY strain were independently cultivated in 50 ml YPAD (2% glucose) on 30°C for 3-5 hours in a shaking incubator (200 rpm) until the exponential phase is reached. Following a washing step with 5ml LiAc (0.1 M), yeast cells were resuspended in 100 µl LiAc (0.1 M) and incubated for 10 min at room temperature before aliquoting. To every aliquot, 1 µg CRISPR plasmid targeting the ARS208a locus, 10 µg purified linear yERα mutant library, 40 µg single-stranded carrier DNA and 750 µl PL solution (42% PEG 3350, 120 mM LiAc) was added. The cell suspensions were incubated for 30 min at 30°C before delivering the 14 min heat shock at 42°C. After centrifugation (3000 rpm 3min), the yeast pellets were resuspended in CaCl.2H₂O (5 mM) and incubated for 5 min at room temperature. Next, cells were washed twice with YPD (2% glucose) before plating on YPD agar (2% glucose) for overnight incubation on 30°C. The following day, the transformants were replica plated on uracil and histidine depleted minimal medium spiked with tamoxifen (10 µM) and grown for 48-72h on 30°C.

### Characterization

Per evolution round, transformants from 60 independent CRISPR-directed library integrations were pooled. Cells expressing mutant yERas with tamoxifen-induced yemCherry signals greater than the ligand-free control were collected using FACS (BD INFLUX). After observing 200.000 cells for both the non-stimulated and tamoxifen stimulated conditions, the sorting gate was set to maximize the difference in cells falling above the gate between the non-stimulated and tamoxifen activated yERα mutant libraries. This generally resulted in collecting the top 2-3% of the tamoxifen induced yERα mutant libraries. FACS-isolated cells were immediately recovered in SC medium from which 200 cells per batch were plated on uracil and histidine depleted minimal medium spiked with tamoxifen (10 µM). On average, 960 colonies were picked across multiple FACS-isolated cell batches to clonally test stimulation responses. Each clone was incubated overnight in SC medium with oestradiol, tamoxifen or without ligand. After 16-24h, the respective yeCitrine and yemCherry response of each clone with and without tamoxifen or oestradiol was measured on a flow cytometer with high-throughput sampler (Attune NxT Flow Cytometer). The clonal yemCherry fluorescence intensities are normalized by the corresponding yeCitrine signals. The sequences of the top 30 tamoxifen selective- and general improved mutants were determined via Sanger sequencing (Table 2-6).

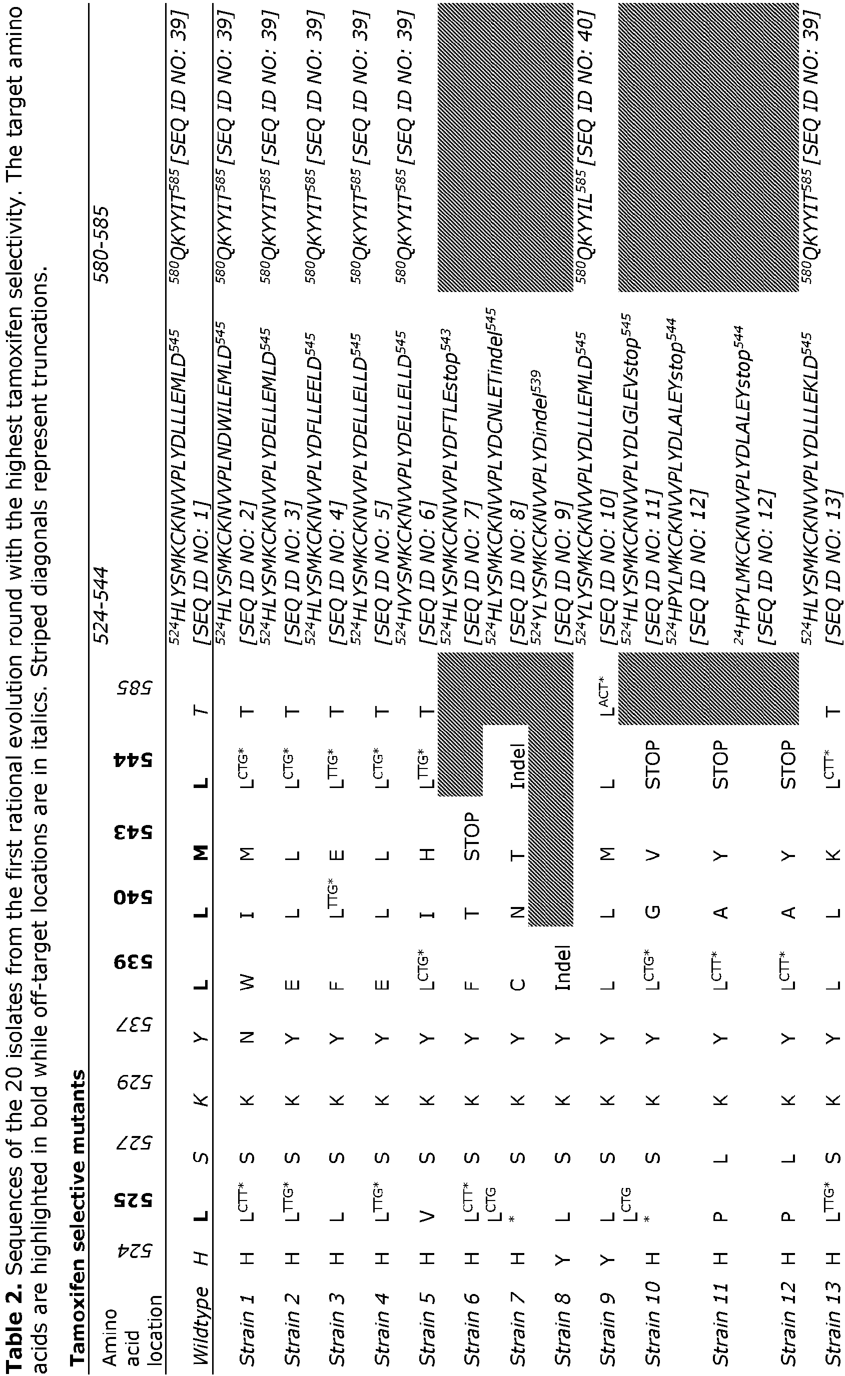

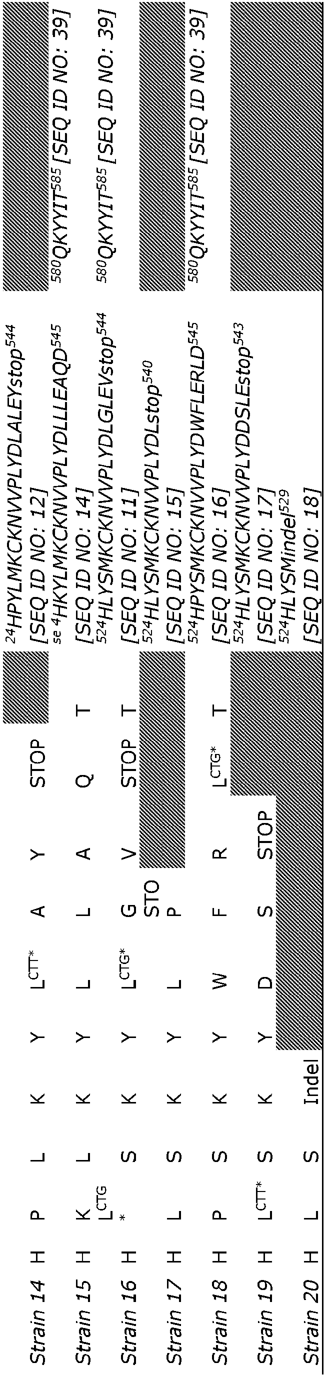

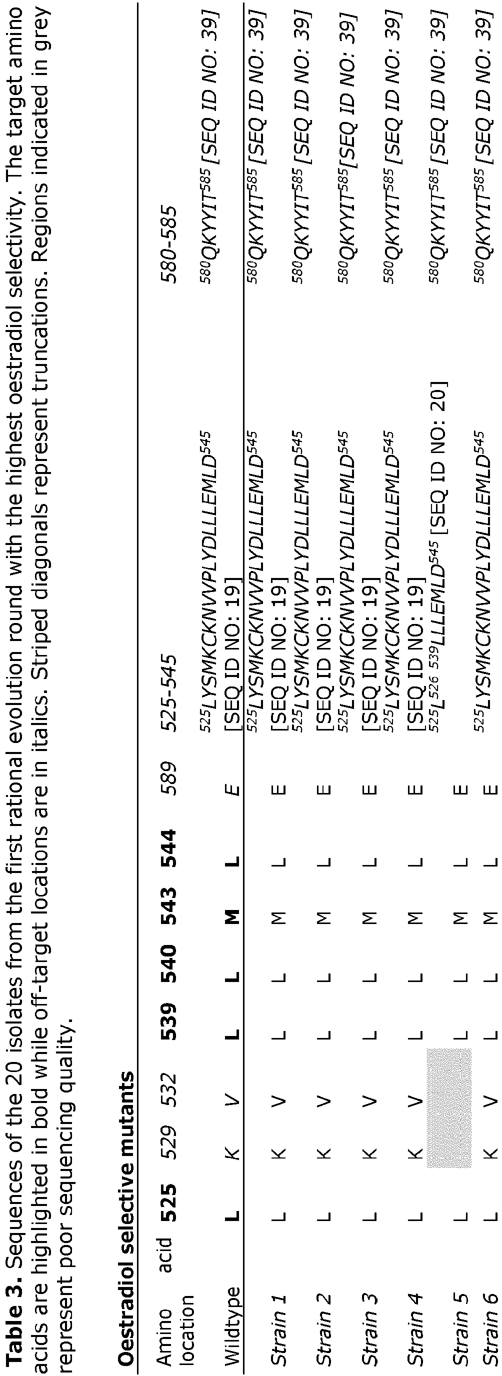

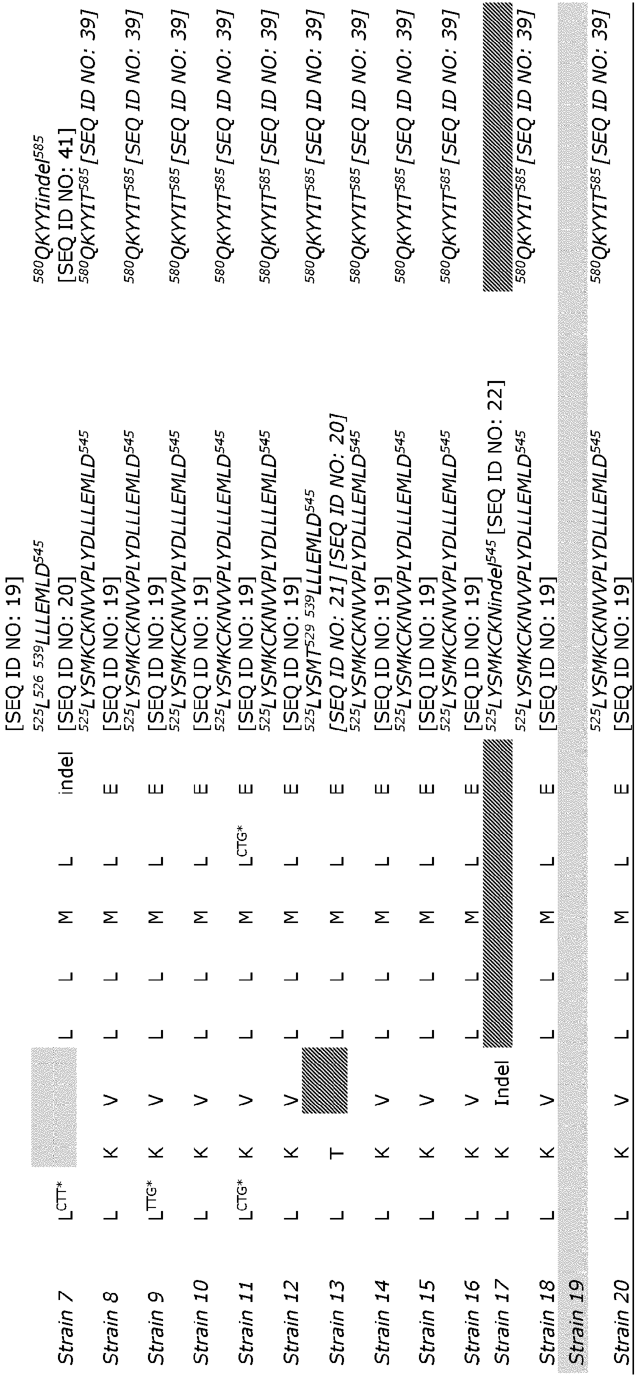

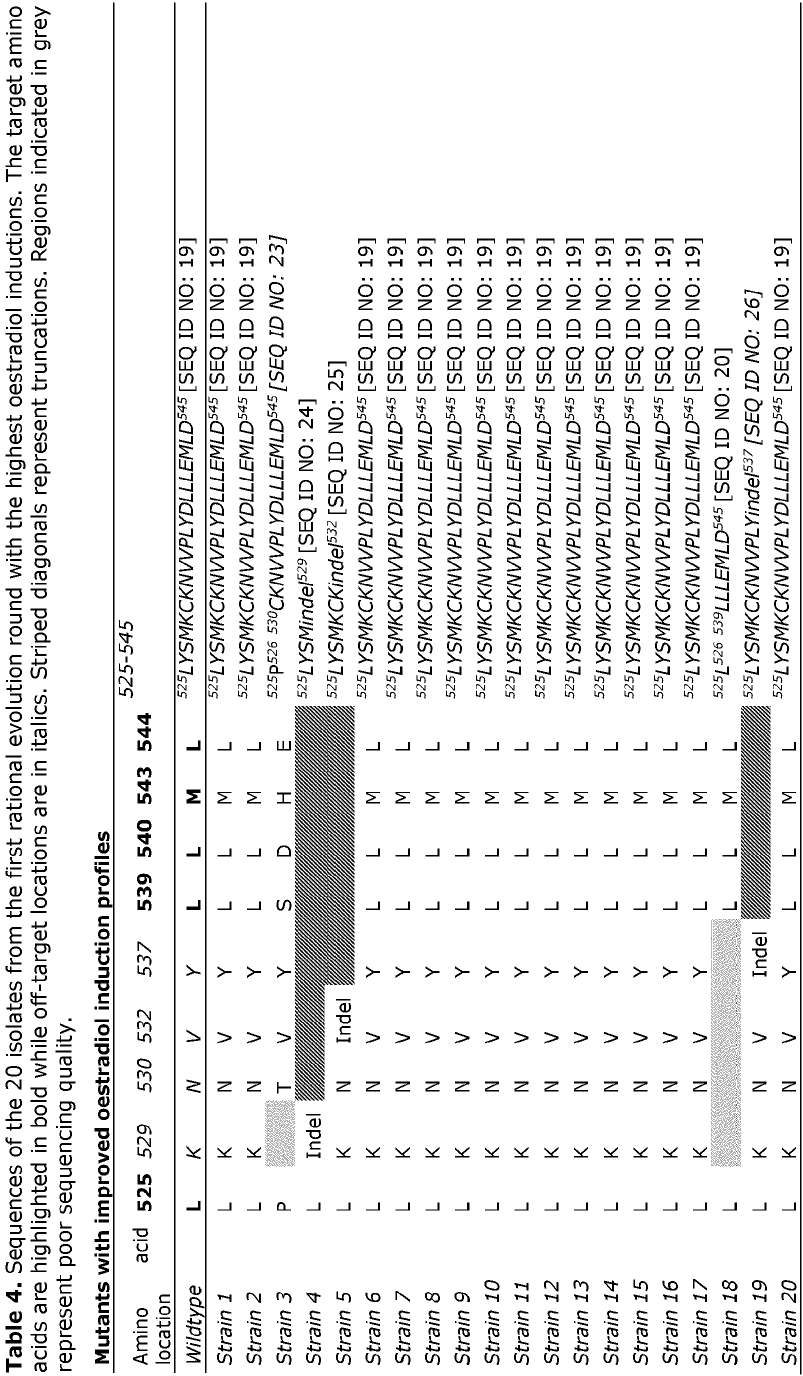

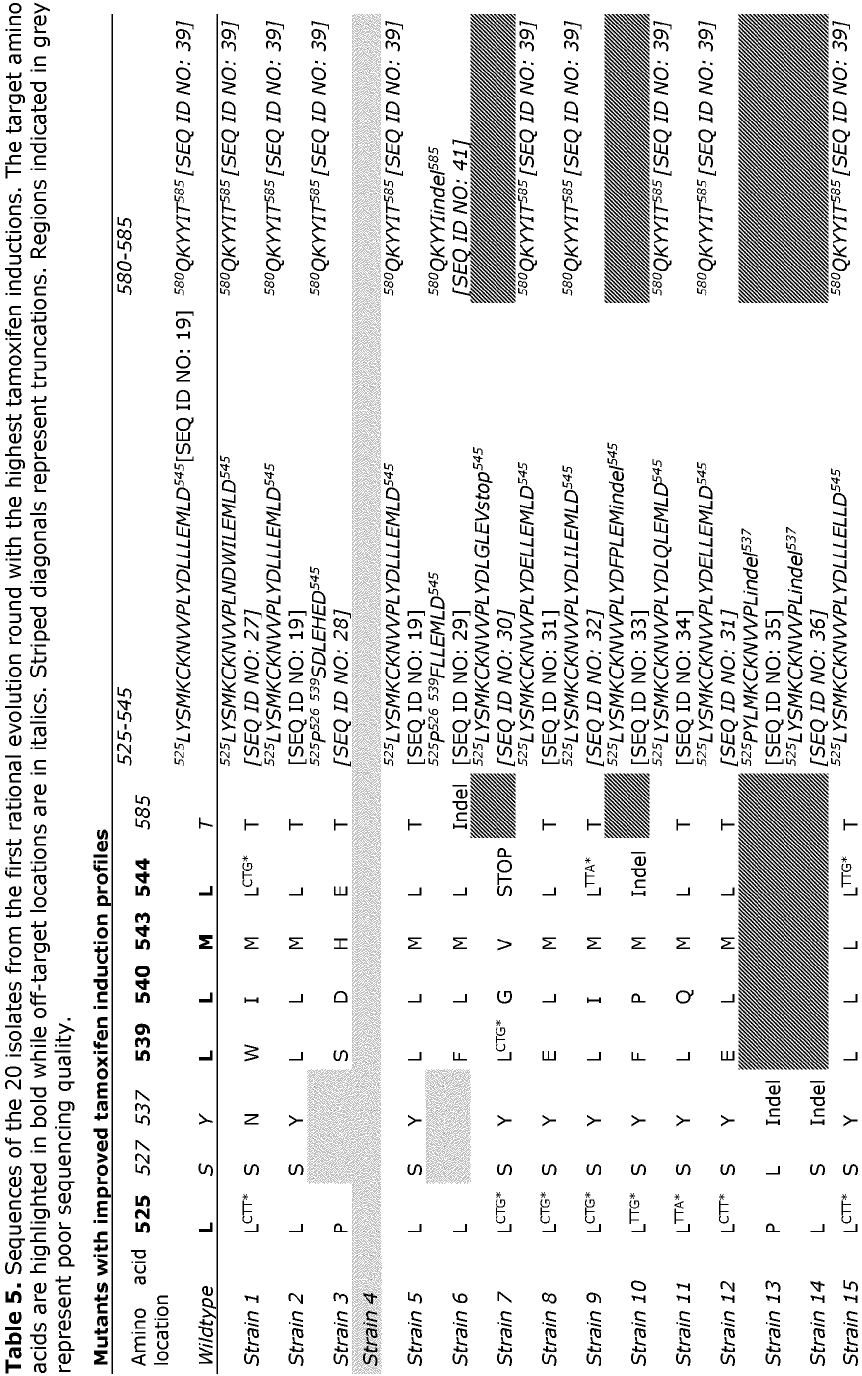

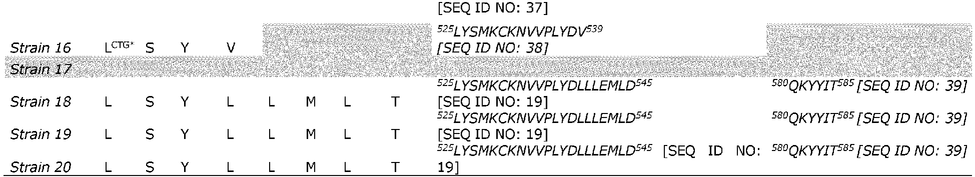

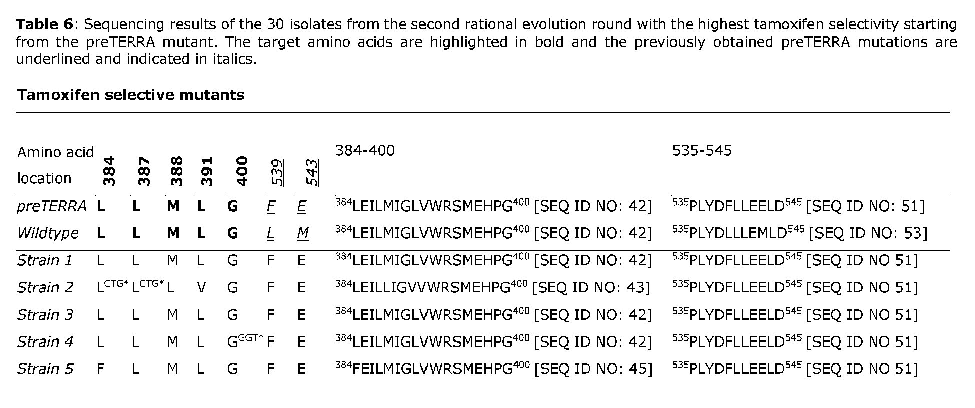

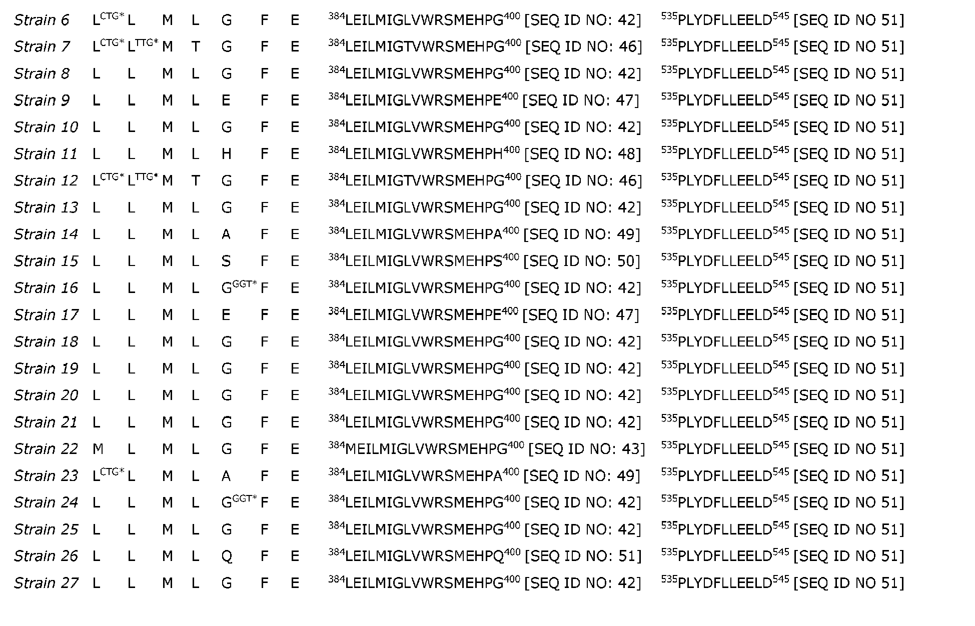

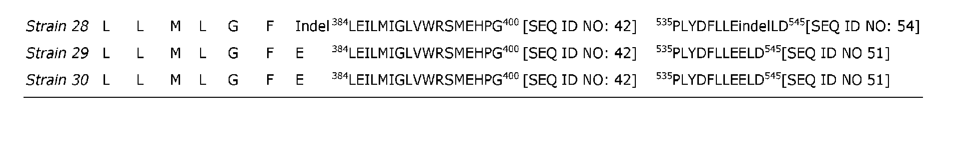

### Mammalian transactivation assays

### Luciferase assay

HEK 293T cells were seeded 24h prior to transfection in a 96-well plate (Greiner) at a density of 15.000 cells per well in DMEM with 5% stripped serum. The seeded cells were transfected with a mixture of 100ng SRi-Luc, 10 ng of hERa or human codon optimized mutant of interest and 5 ng of pCMV-β-gal using GeneJuice^{®} Transfection Reagent conform the manufacturer's instructions. After overnight incubation, the medium was refreshed and spiked with either tamoxifen, oestradiol or vehicle. After 24h stimulation cells were lysed in 25 µl Passive Lysis Buffer (Promega). Luciferase activity and β-galactosidase activity were measured and processed as described²⁷. The observed luciferase activities were corrected by the corresponding β-galactosidase activities.

### Fluorescent assay

HEK293-loxP-GFP-RFP cells were seeded 24h prior to transfection in a 96-well plate (TPP) at a density of 15.000 cells per well in DMEM with 5% stripped serum. The seeded cells were transfected with 1 ng of the Cre-fusion protein of interest, 5 ng of pCMV-β-gal and 109 ng pGEM-T using GeneJuice^{®} Transfection Reagent conform the manufacturer's instructions. After overnight incubation, the medium was refreshed and spiked with either tamoxifen, oestradiol or vehicle. Proliferation and fluorescence (GFP and RFP) were monitored for 110 h with the Incucyte ZOOM live-cell imaging system (Essen Bioscience).

### Mice

### In vivo transduction

Recombinant AAV expressing GFP, Cre-ERT2 or Cre-TERRA were administered via the tail vein to mTmG mice at 5 weeks of age under isoflurane anaesthesia (1.8×10¹² vg/mouse). After 14 days, tamoxifen was administered via oral gavage to induce ERT2 or TERRA. Seven days post tamoxifen-administration, cre recombination activity was assessed by *in vivo* IVIS imaging (TdTomato and GFP) under isoflurane anaesthesia before sacrifice. Liver and hearts were harvested and frozen sections were made for further microscopic analysis.

### Cited references

- 1.: Mangelsdorf et al. Cell 83, 835-839 (1995).
- 2.: Ragavan et al. TrAC Trends in Analytical Chemistry 52, 248-260 (2013).
- 3.: Hettwer et al. Sci Total Environ 621, 612-625 (2018).
- 4.: Kuo et al. PLoS One 7, e50855 (2012).
- 5.: Liang et al. Nucleic Acids Res 41, e54 (2013).
- 6.: Liu et al.Nat Chem Biol 12, 980-987 (2016).
- 7.: Hochrein et al. Nat Commun 9, 1931 (2018).
- 8.: Matsuda & Cepko Proc Natl Acad Sci U S A 104, 1027-1032 (2007).
- 9.: Ciriaco et al. J Pharmacol Pharmacother 4, S94-98 (2013).
- 10.: Tang & Newton J Exp Bot 55, 1499-1508 (2004).
- 11.: Adeel et al. Environ Int 99, 107-119 (2017).
- 12.: Glasgow et al. Science 366, 1024-1028 (2019).
- 13.: Katzenellenbogen et al. Nat Rev Cancer 18, 377-388 (2018).
- 14.: Lu et al. Nat Biotechnol 27, 1139-1150 (2009).
- 15.: Feil et al. Biochem Biophys Res Commun 237, 752-757 (1997).
- 16.: Metzger et al. Proc Natl Acad Sci U S A 92, 6991-6995 (1995).
- 17.: Jordan Oncology (Williston Park) 11, 7-13 (1997).
- 18.: Donocoff et al. Sci Rep 10, 15244 (2020).
- 19.: Xie et al. JBMR Plus 5, e10450 (2021).
- 20.: Kristianto et al. Transgenic Res 26, 411-417 (2017).
- 21.: Pan & Kortemme J Biol Chem 296, 100558 (2021).
- 22.: Marshall et al Drug Discov Today 8, 212-221 (2003).
- 23.: Cozens & Pinheiro Nucleic Acids Res 46, e51 (2018).
- 24.: Pourmir & Johannes Comput Struct Biotechnol J 2, e201209012 (2012).
- 25.: Bulter et al. Appl Environ Microbiol 69, 987-995 (2003).
- 26.: McEwan Trends Genet 17, 239-243 (2001).
- 27.: Eerlings et al. J Steroid Biochem Mol Biol 217, 106043 (2021).
- 28.: Redden & Alper Nat Commun 6, 7810 (2015).
- 29.: Sleight et al. J Biol Eng 4, 12 (2010).
- 30.: Reider Apel et al. Nucleic Acids Res 45, 496-508 (2017).
- 31.: Jardi et al. Mol Cell Endocrinol 452, 57-63 (2017).
- 32.: Kuhlman & Bradley Nat Rev Mol Cell Biol 20, 681-697 (2019).
- 33.: Werstuck & Green Science 282, 296-298 (1998).
- 34.: Taylor et al. Nat Methods 13, 177-183 (2016).
- 35.: DiCarlo et al. Nucleic Acids Res 41, 4336-4343 (2013).
- 36.: Edwards & Wandless Yeast 27, 229-236 (2010).
- 37.: Vyas et al. mSphere 3 (2018).
- 38.: Concordet & Haeussler. Nucleic Acids Res 46, W242-W245 (2018).
- 39.: Mertens, S. et al. PLoS One 14, e0224525 (2019).
- 40.: Gangloff, M. et al. J Biol Chem 276, 15059-15065 (2001).
- 41.: Ashkenazy, H. et al. Nucleic Acids Res 44, W344-350 (2016).
- 42.: Abraham et al. (2015). SoftwareX 1, 19-25

## Claims

1. A polypeptide comprising the ligand binding domain of an oestrogen receptor, **characterised in that** the polypeptide comprises, with reference to the amino acid sequence of human oestrogen receptor isoform 1 [SEQ ID NO: 55], a mutation in the ligand binding domain at position 539 and 543.

2. The polypeptide according to claim 1, which further comprises with reference to the amino acid sequence of human oestrogen receptor isoform 1 [SEQ ID NO: 55], a mutation at position 391.

3. The polypeptide according to claim 1 or 2, wherein the mutation at position 539 is L539F, L539Y or L539W, more particularly the mutation at position 539 is L539F.

4. The polypeptide according to claim 1 or 2, wherein the mutation at position 543 is M543E, M543D, M543Q, or M543N, more particularly the mutation at the mutation at position 543 is M543E.

5. The polypeptide according to claim 1 or 2, wherein the mutation at position 391 is L391T or L931S.

6. The polypeptide according to any one of claims 1 to 5,
wherein the mutation at position 539 is L539F,
wherein the mutation at position 543 is M543E,
and
wherein the mutation at position 391 is L391T.

7. The polypeptide according to any one of claims 1 to 6 which is a fusion protein with a cre-recombinase or a Cas protein.

8. A polynucleotide encoding a polypeptide according to any one of claims 1 to 7.

9. A method of generating modified versions of the ligand binding domain (LBD) of a steroid receptor comprising the steps of:
a) introducing a first set of amino acid mutations at one or more of the amino acids corresponding to the amino acids L525, L539, L540, M543, and L544 of the human oestrogen receptor isoform 1 amino acid sequence [SEQ ID NO: 55],
b) testing a polypeptide comprising the LBD modified in step b) for activation by a compound other than its natural ligand, and selecting mutants which are activated by said compound at an EC50 below 1 µM, or below 500 nM,
c) further introducing a second set of amino acid mutations at one or more of the amino acids corresponding to the amino acids L384, L387, M388, L391, G400 of the human oestrogen receptor isoform 1 amino acid sequence [SEQ ID NO: 55],
d) testing a protein comprising the LBD further modified in step c) for activation by its natural ligand, and selecting mutants which are activated by said natural ligand with an EC50 above 10 nM, above 25 nM, above 50 nM or above 75 nm, or selecting mutants which are activated by said natural ligand at an EC50 which is at least 5, 10, or 20 times higher compared to EC50 obtained by said natural ligand with a non-modified LBD.

10. The method according to claim 9, wherein the EC50 is determined by measuring in a cell the expression level of a steroid dependent reporter gene, or wherein the activity is determined by measuring the fluorescence of a fluorescent reporter protein or by measuring the enzymatic activity of a reporter enzyme.

11. The method according to claim 9, wherein the steroid receptor is the oestrogen receptor, and the natural ligand is oestradiol, and wherein said compound is tamoxifen.

12. The method according to claim 9, wherein the LBD in step c) used for introducing the second set of mutations comprises mutations at position 539 and 543.

13. The method according to claim 9, wherein the LBD in step c) used for introducing the second set of mutations comprises, with reference to the amino acid sequence of human oestrogen receptor isoform 1 amino acid sequence [SEQ ID NO: 55], at position 539 the mutation L539F, L539Y or L539W, and comprises at position 543 the mutation M543D, M543E M543Q, or M543N, more particularly comprises at position 539 the mutation L539F, and comprises at position 543 the mutation M543E.
